# EUROPEAN PATENT SPECIFICATION

(11) **EP 2 120 570 B1**
(45) Date of publication and mention of the grant of the patent: **16.05.2012**
(21) Application number: 08729644.8
(22) Date of filing: 12.02.2008
(51) Int. Cl.: A61K 31/47, A01N 43/32, A61K 31/137, A61K 31/4985, A61K 31/519, A61K 31/53, A61P 15/10

(54) **TREATMENT OF COMORBID PREMATURE EJACULATION AND ERECTILE DYSFUNCTION**
BEHANDLUNG FRÜHZEITIGER COMORBID-EJAKULATION UND EREKTILER DYSFUNKTION
TRAITEMENT DESTINÉ À UNE ÉJACULATION PRÉCOCE ET AUX TROUBLES DE L'ÉRECTION COMORBIDES

(30) Priority: 12.02.2007 US 889369 P
(43) Date of publication of application: 25.11.2009
(73) Proprietor: DMI Biosciences, Inc., Greenwood Village, CO 80111 (US)
(72) Inventor: WINKLER, James, V., Denver, CO 80203 (US); BILYARD, Kevin, Cheshire SK9 5PL (GB); BAR-OR, David, Englewood, CO 80110 (US)
(74) Representative: Thomas, Simon
(86) International application number: PCT/US2008/053710
(87) International publication number: WO 2008/100926

(56) References cited:
- WO-A-2007/070779
- WO-A-2008/100933
- WO-A-2008/131256
- WO-A2-2004/069167
- US-B1- 6 403 597
- US-B2- 6 974 839
- "Vivus' PDE5 inhibitor fails in PE study (Brief Article)", BIOWORLD WEEK,, 7 July 2003 (2003-07-07),
- Statement from Dr Christopher P. Steidle
- Declaraton from inventors filed in proceedings before US Patent and Trademark Office

## Description

### FIELD OF THE INVENTION

The invention relates to the treatment of comorbid premature ejaculation and erectile dysfunction in a human male comprising administering both a tramadol material and a phosphodiesterase inhibitor to the male. The invention specifically relates to a pharmaceutical composition comprising a tramadol material and a phosphodiesterase inhibitor.

### BACKGROUND OF THE INVENTION

Premature ejaculation is the most common form of male sexual dysfunction, with an estimated 22-38% ofmales suffering from it. It is a debilitating sexual dysfunction which can lead to an inability to enter into, or sustain, relationships and can cause psychological damage to sufferers. Premature ejaculation can also impair reproductive success.

Erectile dysfunction is also a common form of male sexual dysfunction, with an estimated 5-15% of males suffering from it. The prevalence of erectile dysfunction increases with age and certain medical conditions, such as heart disease, hypertension and diabetes.

Men often suffer from both premature ejaculation and erectile dysfunction. It has been reported that from about 25 to about 45% of men with premature ejaculation also suffer from erectile dysfunction (Fasolo et al., J. Sex. Med., 2:376-382 (2005); Shabsigh and Perelman, J. Sex Res., Volume 43, Number 1 (February 2006); Porst et al., European Urology , 51(3):816-824 (March 2007; published online July 2006)), and that over half of men with erectile dysfunction also suffer from premature ejaculation (Shabsigh and Perelman, J. Sex Res., Volume 43, Number 1 (February 2006)). Men who suffer from both premature ejaculation and erectile dysfunction have lower scores for quality of life and sexual enjoyment compared to those with either premature ejaculation or erectile dysfunction alone. *Id.*

### SUMMARY OF THE INVENTION

The invention provides a pharmaceutical composition for use in the treatment of comorbid premature ejaculation and erectile dysfunction in a human male to be administered an effective time prior to sexual activity, the composition comprising a pharmaceutically-acceptable carrier and active ingredients, wherein the active ingredients consist of a tramadol material and a phosphodiesterase (PDE) inhibitor.

The invention also provides the use of a composition comprising a pharmaceutically-acceptable carrier and active ingredients, wherein the active ingredients consist of a tramadol material and a phosphodiesterase (PDE) inhibitor, in the preparation of a medicament for the treatment of comorbid premature ejaculation and erectile dysfunction in a human male to be administered an effective time prior to sexual activity.

### BRIEF DESCRIPTION OF THE DRAWINGS

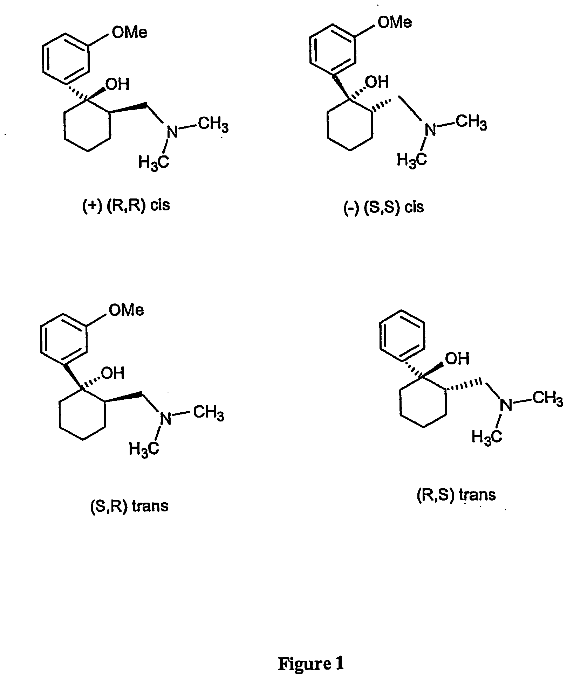

Figure 1 shows stereoisomers of tramadol.

### DETAILED DESCRIPTION OF THE PRESENTLY- PREFERRED EMBODIMENTS OF THE INVENTION

As used herein, the term "premature ejaculation" means a sexual dysfunction wherein a male is unable to control the ejaculatory process to a degree sufficient to satisfy a partner and/or himself. Premature ejaculation refers to persistent or recurring ejaculation with minimal stimulation and/or that occurs sooner than desired, before or shortly after penetration during sexual intercourse, causing distress to one or both partners. See Montague, et al. J. Urol., 172:290-294 (2004); Diagnostic and Statistical Manual of Mental Disorders, 4th ed., American Psychiatric Association, Washington, D.C. (2000). The term includes "congenital," "lifelong," "primary" and "acquired" premature ejaculation.

Although a variety of specific criteria have been proposed for diagnosing premature ejaculation, no one criterion or group of criteria is yet universally accepted. Specific proposed criteria include: (i) ejaculation prior to penetration or within ten to twenty strokes after intromission; (ii) ejaculation in less than 1-2 minutes; and (iii) ejaculation 50% of the time more rapidly than the female is able to have an orgasm if she has no orgasmic dysfunction. See, *e.g.*, U.S. Patent No. 6,037,360 and 5,151,448; Male Infertility and Sexual Dysfunction, page 356 (Springer-Verlag 1997); Diagnostic and Statistical Manual of Mental Disorders (American Psychiatric Association 1994). More recently, an intravaginal ejaculatory latency time ('IELT' or 'IVELT') measured by stopwatch of less than 2 minutes combined with evidence of distress or interpersonal difficulty has been used for the diagnosis of premature ejaculation in clinical studies. See Pryor, et al., Lancet, 368(9539):929-937 (September 9, 2006). One report suggests that men with an IELT of less than 1 minute have "definite" premature ejaculation and that men with an IELT between 1 and 1.5 minutes have "probable" premature ejaculation, whereas the severity of the premature ejaculation (such as "non-symptomatic," "mild," "moderate" and "severe") should be defined in terms of associated psychological problems. Waldinger, et al., J. Sex. Med., 2(4):498-507 (2005). Various self-reported outcome questionnaires, also referred to as patient-reported outcomes, for diagnosing premature ejaculation have been developed. See Althof, et al., Urol. Clin. North Am., 34(4):581-589 (November 2007). The Premature Ejaculation Diagnostic Tool (PEDT) is one such questionnaire. It has recently been validated and indicates that scores of 9 and 10 are "probable" premature ejaculation and scores equal to or greater than 11 are diagnostic of premature ejaculation. See Symonds et al., Eur. Urol., 52:565-573 (2007) and Symonds et al., Int. J. Impot. Res., 19:521-5 (2007) (includes a copy of the questionnaire). This questionnaire evaluates lack of control, frequency of premature ejaculation, minimal sexual stimulation, distress and interpersonal difficulties. Presently, the inventors consider that the best criteria for diagnosing premature ejaculation are a short IELT plus a PEDT score of 9 or greater. The exact definition of a short IELT is expected to vary depending on geographic area and/or cultural differences, and can be determined empirically. For the United States, the inventors presently consider that the best definition of a short IELT is an IELT of less than 2 minutes in greater than 50% of coital attempts as measured using a stopwatch.

As used herein, the term "erectile dysfunction" means the consistent or recurrent inability to achieve and/or maintain a penile erection sufficient to permit satisfactory sexual intercourse or activity. "Erectile dysfunction" is also used herein to mean the partial, temporary or episodic absence of a penile erection.

The erectile function (EF) domain of the International Index of Erectile Function (IIEF) has been developed and validated as a patient-based questionnaire that is now widely use for the diagnosis of erectile dysfunction. The EF domain of the IIEF has demonstrated test reliability and validity with a high degree of sensitivity and specificity. In particular, men scoring 25 or less are classified as having ED and those scoring above 25 are classified as not having ED (sensitivity = 0.97; specificity = 0.88). Further, responses to IIEF erectile function questions can classify erectile dysfunction into five diagnostic categories: no erectile dysfunction (score 26-30); 'mild' erectile dysfunction (score 22-25); 'mild-to-moderate' erectile dysfunction (score 17-21); 'moderate' erectile dysfunction (score 11-16); and 'severe' erectile dysfunction (score 6-10). See Rosen, et al., Int. J. Impot. Res., 14(4):226-244 (August 2002) and Rosen, et al., Urology, 49:822-830 (1997) (includes a copy of the IIEF questionnaire and an identification of the EF questions). Another subset of the IIEF called the Sexual Health Inventory for Men (SHIM) was also developed and validated as a diagnostic tool that is now widely use for the diagnosis of erectile dysfunction. Men scoring 21 or less are classified as having ED and those scoring above 21 are classified as not having ED (sensitivity = 0.98; specificity = 0.88). Further, responses to SHIM questions can classify erectile dysfunction into five diagnostic categories: no erectile dysfunction (score 22-25); 'mild' erectile dysfunction (score 17-21); 'mild-to-moderate' erectile dysfunction (score 12-16); 'moderate' erectile dysfunction (score 8-11); and 'severe' erectile dysfunction (score 5-7). See Rosen, et al., Int. J. Impot. Res., 14(4):226-244 (August 2002) (identifies the SHIM questions) and Rosen, et al., Urology, 49:822-830 (1997) (includes a copy of the IIEF questionnaire). Accordingly, erectile dysfunction can be diagnosed using the EF score and/or the SHIM score, and the severity of erectile dysfunction can also be assessed using these scores.

As used herein, the term "comorbid" means that two diseases coexist or are found in the same person. "Comorbid premature ejaculation and erectile dysfunction" means that premature ejaculation and erectile dysfunction coexist or are found in the same human male, *i.e*, that a single human male experiences, tends to experience, or has a history of experiencing, both premature ejaculation and erectile dysfunction.

The term "tramadol material" is used herein to refer to 2-[(dimethylamino)methyl]-1-(3-methoxyphenyl)-cyclohexanol ("tramadol") and all pharmaceutically-acceptable forms and derivatives of tramadol. In particular, the term includes the N-oxide derivative ("tramadol N-oxide") and the O-desmethyl derivative ("O-desmethyl tramadol"). The term also includes the solvates, polymorphs, and pharmaceutically-acceptable acid addition salts of tramadol and its derivatives. The term further includes all of the stereoisomers of any of the foregoing, including individual stereoisomers (including individual enantiomers) and mixtures of stereoisomers (including the racemates).

The stereoisomers of tramadol are shown in Figure 1. There appears to be some discrepancy in the literature regarding the nomenclature of the individual stereoisomers of tramadol. For the purposes of the present application, the designations of "cis" and "trans" stereoisomers of tramadol are made in reference to the relative positions of the dimethylamino and the hydroxy substituents on the cyclohexane ring within the tramadol molecule. As shown in Figure 1, the R,R and S,S enantiomers will be referred to herein as the "cis" isomers while the R,S and S,R isomers will be referred to herein as the "trans" isomers. As also shown in Figure 1, the R,R isomer of tramadol will be referred to herein as the "+" cis isomer and the S,S isomer will be referred to as the "-" cis isomer. It is presently understood that R,S and S,R isomers are not optically active.

Presently preferred is tramadol and the acid addition salts thereof, particularly the hydrochloride. Even more preferred is (±)cis-tramadol, the acid addition salts, particularly the hydrochloride, and the individual enantiomers.

Methods of making tramadol, tramadol N-oxide, and O-desmethyl tramadol are well known. See, e.g., U.S. Patents Nos. 3,652,589, 3,830,934, 5,223,541, 5,336,691, 5,723,668, 5,728,885, and 5,874,620, the complete disclosures of which are incorporated herein by reference. Tramadol is also commercially available from several sources, including Gruenenthal GmbH, Aschen, Germany.

The pharmaceutically-acceptable acid addition salts are prepared by conventional methods well known in the art using pharmaceutically-acceptable, substantially non-toxic, organic and inorganic acids. Such acids include hydrochloric acid, nitric acid, sulfuric acid, phosphoric acid, hydrobromic acid, acetic acid, propionic acid, maleic acid, malonic acid, succinic acid, citric acid, tartaric acid, malic acid, benzoic acid, salicylic acid, phthalic acid, nicotinic acid, etc. Preferred is hydrochloric acid, and tramadol hydrochloride is the most preferred compound for practicing the invention.

Phosphodiesterases (PDEs) are a class of intracellular enzymes involved in the metabolism of the second messenger nucleotides cAMP and cGMP. The PDEs have now been classified into eleven major families, Types I-XI. The members of the families vary in their tissue, cellular and subcellular distribution, as well as their links to the cAMP and cGMP pathways. For example, PDE type III (PDE3), PDE type IV (PDE4) and PDE type V (PDE5) are found in the corpus cavemosum, with PDE5 being the most abundant.

A "phosphodiesterase inhibitor" is an agent that is capable of inhibiting or reducing, selectively or nonselectively, the activity of a PDE. Suitable PDE inhibitors for use in the present invention include those described in U.S. Patents Nos. 5,250,534, 5,859,006, 6,140,329, 6,362,178, 6,403,597, 6,469,012, 6,821,975, 6,943,166 and 6,943,171, the complete disclosures of which are incorporated herein by reference. Methods of making PDE inhibitors are known. See, *e.g.*, U.S. Patents Nos. 5,250,534, 5,859,006, 6,140,329, 6,362,178, 6,403,597, 6,469,012, 6,821,975, 6,943,166 and 6,943,171. The PDE inhibitor used in the present invention is preferably an inhibitor of PDE3, PDE4 and/or PDE5. More preferably, the PDE inhibitor is a selective inhibitor of PDE5. Even more preferably, the inhibitor is sildenafil, vardenafil and/or tadalafil, and pharmaceutically-acceptable forms (*e.g.*, salts, solvates, stereoisomers (individual isomers and mixtures of isomers), etc.) of them. Most preferably, the inhibitor is sildenafil citrate (*e.g.*, Viagra® sildenafil citrate; Pfizer), vardenafil hydrochloride (*e.g.*, Levitra® vardenafil HCl; Schering-Plough) and/or tadalafil (*e.g.*, Cialis®; Lilly ICOS).

To treat comorbid premature ejaculation and erectile dysfunction, an effective amount of a tramadol material and an effective amount of a phosphodiesterase inhibitor are administered to a male an effective time prior to sexual activity. The two drugs are administered simultaneously in combination in a single dosage form by a single route of administration. By an "effective amount" is meant a nontoxic, but sufficient, amount of each of the two drugs to delay ejaculation and to reduce the incidence or severity of erectile dysfunction. By an "effective time" is meant the range of time prior to sexual activity during which each of the two drugs must be administered so that they will be effective to delay ejaculation and reduce the incidence of erectile dysfunction. An effective amount of (±)cis-tramadol HCl is from about 1 mg to about 250 mg, preferably from about 10 mg to about 200 mg, more preferably from about 25 mg to about 150 mg, given orally from about 30 minutes to about 24 hours before sexual activity. An effective amount of Viagra® sildenafil citrate is from about 5 mg to about 500 mg, preferably from about 25 mg to about 100 mg, given orally from about 30 minutes to about 4 hours before sexual activity. An effective amount of Levitra® vardenafil hydrochloride is from about 1 mg to about 100 mg, preferably from about 5 mg to about 20 mg, given orally from about 30 minutes to about 2 hours before sexual activity. An effective amount of Cialis® tadalafil is from about 1 mg to about 100 mg, preferably from about 5 mg to about 20 mg, given orally from about 30 minutes to about 36 hours before sexual activity. However, it is understood by those skilled in the art that the dosage amount will vary with the particular form of tramadol employed and the particular phosphodiesterase inhibitor employed, the route(s) of administration, the timing of the administration, the identity of any other drugs being administered, the severity of the premature ejaculation and erectile dysfunction conditions, the age, size and condition of the patient, and like factors known in the medical art. In general, a suitable dose will be that amount of the compound which is the lowest dose effective to delay ejaculation and reduce the incidence or severity of erectile dysfunction without toxicity. However, the dosage, route of administration, etc., will be determined by an attending physician within the scope of sound medical judgment. Effective dosage forms, modes and times of administration, and dosage amounts can be determined empirically.

As used herein, "delay ejaculation" means that a male receiving treatment is able to control the ejaculatory process so as to prevent ejaculation for a time which is longer than that normally experienced by the male when not receiving treatment. It is expected that the male will be able to control the ejaculatory process to a degree sufficient to better or completely satisfy his partner. "Delay ejaculation" does not mean to totally prevent ejaculation.

As used herein, "reduce the incidence of erectile dysfunction" means that erectile dysfunction will be prevented in a male receiving treatment according to the invention or that the number of incidences of erectile dysfunction will be reduced. As used herein, "reduce the severity of erectile dysfunction" means that the severity of erectile dysfunction as measured by the EF and/or SHIM score is reduced (*i.e*., the EF or SHIM score increases).

The tramadol material and phosphodiesterase inhibitor may be administered by any suitable route of administration, including orally, nasally, rectally, parenterally (*e.g.*, intravenously, subcutaneously, or intramuscularly), topically (*i.e.*, delivery to the skin or mucosa), transdermally (*i.e.*, delivery by passage of a drug through the skin into the bloodstream), transmucosally (*i.e.*, delivery by passage of a drug through the mucosal tissue into the bloodstream), intracavernosally (*i.e.*, injection into one or both corpora of the corpora cavemosal tissues of the penis), and intarurethrally (*i.e.*, delivery into the urethra). Highly preferred is oral administration.

The tramadol material and phosphodiesterase inhibitor are administered in combination as a pharmaceutical formulation (composition). The pharmaceutical compositions may comprise a tramadol material and a phosphodiesterase inhibitor as the active ingredients in admixture with one or more pharmaceutically-acceptable carriers and, optionally, with one or more other compounds, or other materials. Each carrier must be "acceptable" in the sense of being compatible with the other ingredients of the formulation and not injurious to the male who will take the composition. Pharmaceutically-acceptable carriers are well known in the art. Regardless of the route of administration selected, the active ingredient are formulated into pharmaceutically-acceptable dosage forms by conventional methods known to those of skill in the art. See, *e.g., Remington's Pharmaceutical Sciences*

Pharmaceutical compositions containing a tramadol material and methods of making the pharmaceutical compositions have been described. See, e.g., U.S. Patents Nos. 3,652,589, 3,830,934, 5,223,541, 5,591,452, 5,601,842, 5,728,885, 6,017,963, 6,090,856, and 6,156,342, the complete disclosures of which are incorporated herein by reference. Moreover, pharmaceutical compositions containing tramadol and pharmaceutically-acceptable salts thereof are manufactured and sold worldwide. In the United States, (±) *cis*-2-[(dimethylaminomethyl]-1-(3-methoxyphenyl)-cyclohexanol hydrochloride for oral administration is available from Ortho-McNeil Pharmaceutical, Inc., Raritan, New Jersey 08869, as ULTRAM tablets. Each ULTRAM tablet contains 50 mg (±) *cis-2-*[(dimethylamino)methyl]-1-(3-methoxyphenyl)-cyclohexanol hydrochloride and a number of inactive ingredients (corn starch, hydroxypropyl methylcellulose, lactose, magnesium stearate, microcrystalline cellulose, polyethylene glycol, polysorbate 80, sodium starch glycolate, titanium dioxide and wax). It is understood that the commercial preparation of tramadol marketed under the brand name ULTRAM® consists of a mixture of the R,R and S,S isomers of tramadol hydrochloride.

Pharmaceutical compositions containing a phosphodiesterase inhibitor and methods of making the pharmaceutical compositions have also been described. See, *e.g.*, U.S. Patents Nos. 5,250,534, 5,859,006, 6,140,329, 6,362,178, 6,403,597, 6,469,012, 6,821,975, 6,943,166 and 6,943,171, the complete disclosures of which are incorporated herein by reference. Suitable phosphosdiesterase inhibitors are also available commercially from, *e.g*, Pfizer (Viagra® sildenafil citrate), Schering-Plough (Levitrac® vardenafil HCl) and Lilly ICOS (Cialis® tadalafil).

Formulations of the invention suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of the active ingredients. Preferred oral administration forms are tablets and capsules.

In solid dosage forms of the invention for oral administration (capsules, tablets, pills, dragees, powders, granules and the like), the active ingredients are mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin orhydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredients therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredients only, or preferentially, in a certain portion of the gastrointestinal tract, optionally, in a delayed manner. These compositions may also be of a composition so that they release the active ingredients only, or preferentially, in a certain sequence (*e.g.*, one before the other, one immediately and the other over time, both over time but with different release profiles, etc.). Examples of embedding compositions which can be used include polymeric substances and waxes. The active ingredients can also be in microencapsulated form.

Liquid dosage forms for oral administration of the compounds of the invention include pharmaceutically-acceptable emulsions, microemulsions, solutions, suspensions, syrups and elixirs. In addition to the active ingredients, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming, thickening, and preservative agents.

Suspensions, in addition to the active ingredients, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal administration may be presented as a suppository, which may be prepared by mixing the active ingredients with one or more suitable nonirritating excipients or carriers comprising, for example, cocoa butter, polyethylene glycol, a suppository wax or salicylate, and which is solid at room temperature, but liquid at body temperature and, therefore, will melt in the rectum and release the active ingredients.

Dosage forms for the topical, transdermal or transmucosal administration of the active ingredients include powders, sprays, ointments, pastes, creams, lotions, gels, solutions, patches, drops and inhalants. The active ingredients may be mixed under sterile conditions with a pharmaceutically-acceptable carrier, and with any buffers, or propellants which may be required.

The ointments, pastes, creams and gels may contain, in addition to the active ingredients, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the active ingredients, excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane.

The active ingredients may also be delivered through the skin using conventional transdermal drug delivery systems, *i.e.*, transdermal patches, wherein the active ingredients are typically contained within a laminated structure that serves as a drug delivery device to be affixed to the skin. In such a structure, the active ingredients are typically contained in a layer, or "reservoir," underlying an upper backing layer. The laminated device may contain a single reservoir, or it may contain multiple reservoirs. In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form.

The backing layer in these laminates, which serves as the upper surface of the device, functions as the primary structural element of the laminated structure and provides the device with much of its flexibility. The material selected for the backing material should be selected so that it is substantially impermeable to the active ingredient and any other materials that are present. The backing layer may be either occlusive or nonocclusive, depending on whether it is desired that the skin become hydrated during drug delivery. The backing is preferably made of a sheet or film of a preferably flexible elastomeric material. Examples of polymers that are suitable for the backing layer include polyethylene, polypropylene, polyesters, and the like.

During storage and prior to use, the laminated structure includes a release liner. Immediately prior to use, this layer is removed from the device to expose the basal surface thereof, either the drug reservoir or a separate contact adhesive layer, so that the system may be affixed to the skin. The release liner should be made from a drug/vehicle impermeable material.

Transdermal drug delivery devices may be fabricated using conventional techniques, known in the art, for example by casting a fluid admixture of adhesive, drug and vehicle onto the backing layer, followed by lamination of the release liner. Similarly, the adhesive mixture may be cast onto the release liner, followed by lamination of the backing layer. Alternatively, the drug reservoir may be prepared in the absence of drug or excipient, and then loaded by "soaking" in a drug/vehicle mixture.

The laminated transdermal drug delivery systems may in addition contain a skin permeation enhancer. That is, because the inherent permeability of the skin to some drugs may be too low to allow therapeutic levels of the drug to pass through a reasonably sized area of unbroken skin, it is necessary to coadminister a skin permeation enhancer with such drugs. Suitable enhancers are well known in the art.

The pharmaceutical compositions of the invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, propellants such as fluorocarbons or nitrogen, and/or other conventional solubilizing or dispersing agents.

Preferred formulations for topical drug delivery are ointments and creams. Ointments are semisolid preparations which are typically based on petrolatum or other petroleum derivatives. Creams containing the selected active agents, are, as known in the art, viscous liquid or semisolid emulsions, either oil-in-water or water-in-oil. Cream bases are water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. The specific ointment or cream base to be used, as will be appreciated by those skilled in the art, is one that will provide for optimum drug delivery. As with other carriers or vehicles, an ointment base should be inert, stable, nonirritating and nonsensitizing.

Formulations for buccal administration include tablets, lozenges, gels and the like. Alternatively, buccal administration can be effected using a transmucosal delivery system as known to those skilled in the art.

Pharmaceutical compositions suitable for parenteral administrations comprise the active ingredients in combination with one or more pharmaceutically-acceptable sterile isotonic aqueous or non-aqueous solutions, dispersions, suspensions or emulsions, or sterile powders or other solid forms which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of injectable pharmaceutical forms may be brought about by the inclusion of agents which delay absorption such as aluminum monosterate and gelatin.

In some cases, in order to prolong the effect of the active ingredients, it is desirable to slow the absorption of the active ingredients from subcutaneous or intramuscular injection. This may be accomplished by the use of a liquid suspension of crystalline or amorphous material having poor water solubility. The rate of absorption of the active ingredients then depends upon its rate of dissolution which, in turn, may depend upon crystal size and crystalline form. Alternatively, delayed absorption of a parenterally-administered active ingredients is accomplished by dissolving or suspending the drug in an oil vehicle.

Injectable depot forms are made by forming microencapsule matrices of the active ingredients in biodegradable polymers such as polylactide-polyglycolide. Depending on the ratio of active ingredients to polymer, and the nature of the particular polymer employed, the rate of release of the active ingredients can be controlled. Examples of other biodegradable polymers include poly(orthoesters) and poly(anhydrides). Depot injectable formulations are also prepared by entrapping the active ingredients in liposomes or microemulsions which are compatible with body tissue. The injectable materials can be sterilized for example, by filtration through a bacterial-retaining filter.

Intracavernosal injection can be carried out by use of a syringe or any other suitable device. An example of a hypodermic syringe useful herein, that can be used for simultaneous injection into both corpora, is described in U.S. Pat. No. 4,127,118. The injection is made on the dorsum of the penis by placement of the needle to the side of each dorsal vein and inserting it deep into the corpora.

The active ingredients can be administered in a pharmaceutical formulation suitable for transurethral drug delivery. The formulation contains one or more selected carriers or excipients, such as water, silicone, waxes, petroleum jelly, polyethylene glycol, propylene glycol, liposomes, sugars such as mannitol and lactose, and/or a variety of other materials, with polyethylene glycol and derivatives thereof particularly preferred. It may be desirable to incorporate a transurethral permeation enhancer in the urethral dosage form. Examples of suitable transurethral permeation enhancers include dimethylsulfoxide, dimethyl formaminde, N,N-dimethylacetamide, decylmethylsulfoxide, polyethylene glycol monolaurate, glycerol monolaurate, lecithin, the 1-substituted azacycloheptan-2-ones, particularly 1-n-dodecylcyclazacycloheptan-2-one (available under the trademark Azone^{®} from Nelson Research & Development Co., Irvine, Calif.), SEPA^{®} (available from Macrochem Co., Lexington, Mass.), alcohols (*e.g.*, ethanol), detergents (such as Tergitol^{®}, Nonoxynol-9^{®} and TWEEN-80^{®}) and the like. Transurethral formulations may additionally include one or more enzyme inhibitors effective to inhibit drug-degrading enzymes which may be present in the urethra. Additional optional components include excipients, preservatives (*e.g.*, antioxidants), chelating agents, solubilizing agents (*e.g.*, surfactants), and the like, as will be appreciated by those skilled in the art of drug formulation preparation and delivery.

Transurethral drug administration, as explained in PCT application WO 91/16021, can be carried out in a number of different ways using a variety of urethral dosage forms. For example, the drug can be introduced into the urethra from a flexible tube, squeeze bottle, pump or aerosol spray. The drug may also be contained in coatings, pellets or suppositories which are absorbed, melted or bioeroded in the urethra. In certain embodiments, the drug is included in a coating on the exterior surface of a penile insert. Drug delivery devices for administering a drug transurethrally are described in U.S. Patent No. 6,037,360 and PCT application WO 91/16021.

Urethral suppository formulations containing polyethylene glycol or a polyethylene glycol derivative can be used as the urethral dosage form, and may be conveniently formulated using conventional techniques, *e.g.*, compression molding, heat molding or the like, as will be appreciated by those skilled in the art and as described in the pertinent literature and pharmaceutical texts. See, for example, Remington: The Science and Practice of Pharmacy, 19th Ed. (Easton, PA: Mack Publishing Co., 1995), which discloses typical methods of preparing pharmaceutical compositions in the form of urethral suppositories. It is also preferred that urethral suppositories contain one or more solubilizing agents (*e.g.*, a nonionic, anionic, cationic or amphoteric surfactant) effective to increase the solubility of the active ingredients in the polyethylene glycol or other transurethral vehicle.

It may be desirable to deliver the active ingredients in a urethral dosage form which provides for controlled or sustained release of the agents. In such a case, the dosage form typically comprises a biocompatible, biodegradable material, typically a biodegradable polymer. Examples of such polymers include polyester, polyalkylcyanoacrylate, polyorthoester, polyanhydride, albumin, gelatin and starch. As explained, for example, in PCT application WO 96/40054, these and other polymers can be used to provide biodegradable microparticles which enable controlled and sustained drug release, in turn minimizing the required dosing frequency.

The method of intraurethral administration may involve an "active" delivery mechanism such as iontophoresis, electroporation or phonophoresis. Devices and methods for delivering drugs in this way are well known in the art. Iontophoretically assisted drug delivery is, for example, described in PCT application WO 96/40054. Briefly, the active agents are driven through the urethral wall by means of an electric current passed from an external electrode to a second electrode contained within or affixed to a urethral probe.

The pharmaceutical formulations may be presented in unit-dose or multi-dose sealed containers, for example, ampules, vials and blister packs, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

The invention also provides a kit (not according to the invention) comprising a tramadol material and a phosphodiesterase inhibitor. The kit may comprise one or more containers, each of which contains a tramadol material and a phosphodiesterase inhibitor. In such a case, the tramadol material and the phosphodiesterase inhibitor are preferably contained in the same pharmaceutical composition. Alternatively, the kit may comprise a container holding the tramadol material and a different container holding the phosphodiesterase inhibitor. Suitable containers include tubes, ampules, vials, bottles, foil packets, the wells of a tray and the molded depressions of blister packs. The kit will also comprise instructions for administration of the tramadol material and the phosphodiesterase inhibitor to treat comorbid premature ejaculation and erectile dysfunction. The container(s) will preferably be contained in a package, such as a box. The instructions may be attached to, or printed on, one of the containers, may be printed on a separate sheet of paper inside the package, or may be attached to or printed on the package.

It is to be noted that "a" or "an" entity refers to one or more of that entity. For example, "a container" refers to one or more containers.

### EXAMPLES

### Example 1

A 51-year-old heterosexual male with a long history of premature ejaculation and erectile dysfunction was evaluated by a urologist who determined his premature ejaculation was 'severe' (documented intravaginal ejaculatory latency by stopwatch of less than 1 minute and Premature Ejaculation Diagnostic Tool (PEDT) score of 15) and co-morbid with 'mild to moderate' erectile dysfunction (International Index of Erectile Function (IIEF) erectile function (EF) score of 21).

The subject was initially treated with selective serotonin reuptake inhibitor (SSRI) medications; however, SSRI treatment was only partially effective in treating the premature ejaculation and did not improve his erectile dysfunction. The subject discontinued all SSRI drugs due to low efficacy and intolerable side effects. Sildenafil citrate (Viagra®) 50mg administered before intercourse somewhat improved the erectile dysfunction but did not relieve the severe premature ejaculation, which continued to cause significant emotional distress for the subject and his sexual partner.

At the confidential suggestion of one of the inventors of the present invention, the urologist subsequently prescribed tramadol HCl with instructions to self administer a combination of tramadol HCl 100mg and sildenafil citrate 50mg approximately 3 hours before intercourse. The results have been described as 'miraculous' with the subject's only complaint being excessively delayed ejaculation ('much greater than 10 minutes') and occasional anorgasmia. These moderate adverse events of excessively delayed ejaculation and anorgasmia were not permanent and were no longer reported after reducing the tramadol HCl dose to 50mg. The subject now reports that this combination 'works dramatically well' and his co-morbid condition of premature ejaculation and erectile dysfunction is successfully treated with a combination of tramadol HCl 50mg and sildenafil citrate 50mg administered before intercourse.

## Claims

1. A pharmaceutical composition for use in the treatment of comorbid premature ejaculation and erectile dysfunction in a human male to be administered an effective time prior to sexual activity, the composition comprising a pharmaceutically-acceptable carrier and active ingredients, wherein the active ingredients consist of a tramadol material and a phosphodiesterase (PDE) inhibitor.

2. The composition for use according to claim 1, wherein the tramadol material is tramadol or a pharmaceutically-acceptable form thereof, and the PDE inhibitor is a PDE3, PDE4 or PDE5 inhibitor.

3. The composition for use according to claim 2, wherein the PDE inhibitor is a PDE5 inhibitor.

4. The composition for use according to claim 3, wherein the tramadol material is (±)cis-tramadol or a pharmaceutically-acceptable salt thereof, and the PDE5 inhibitor is sildenafil or a pharmaceutically-acceptable form thereof vardenafil or a pharmaceutically-acceptable form thereof, tadalafil or a pharmaceutically-acceptable form thereof, or a combination of two or more of the foregoing PDE5 inhibitors.

5. The composition for use according to claim 4, wherein the tramadol material is (±)cis-tramadol hydrochloride, and the PDE5 inhibitor is sildenafil citrate, vardenafil hydrochloride, tadalafil or a combination of two or more of the foregoing PDE5 inhibitors.

6. The composition for use according to claim 5, wherein the PDE5 inhibitor is sildenafil citrate.

7. Use of a composition comprising a pharmaceutically-acceptable carrier and active ingredients, wherein the active ingredients consist of a tramadol material and a phosphodiesterase (PDE) inhibitor, in the preparation of a medicament for the treatment of comorbid premature ejaculation and erectile dysfunction in a human male to be administered an effective time prior to sexual activity.

8. The use of claim 7, wherein the tramadol material is tramadol or a pharmaceutically-acceptable form thereof, and the PDE inhibitor is a PDE3, PDE4 or PDE5 inhibitor.

9. The use of claim 8, wherein the PDE inhibitor is a PDE5 inhibitor.

10. The use of claim 9, wherein the tramadol material is (±)cis-tramadol or a pharmaceutically-acceptable salt thereof, and the PDE5 inhibitor is sildenafil or a pharmaceutically-acceptable form thereof, vardenafil or a pharmaceutically-acceptable form thereof, tadalafil or a pharmaceutically-acceptable form thereof, or a combination of two or more of the foregoing PDE5 inhibitors.

11. The use of claim 10, wherein the tramadol material is (±)cis-tramadol hydrochloride, and the PDE5 inhibitor is sildenafil citrate, vardenafil hydrochloride, tadalafil or a combination of two or more of the foregoing PDE5 inhibitors.

12. The use of claim 11, wherein the PDE5 inhibitor is sildenafil citrate.

## Patentansprüche

1. Pharmazeutische Zusammensetzung zur Verwendung bei der Behandlung von komorbidem vorzeitigem Samenerguss und erektiler Dysfunktion bei einem Mann, die eine wirksame Zeit vor sexueller Aktivität zu verabreichen ist, wobei die Zusammensetzung einen pharmazeutisch unbedenklichen Trägerstoff und Wirkstoffe umfasst, wobei die Wirkstoffe aus einem Tramadol-Material und einem Phosphodiesterase-Hemmer (PDE-Hemmer) bestehen.

2. Zusammensetzung zur Verwendung nach Anspruch 1, wobei das Tramadol-Material Tramadol oder eine pharmazeutisch unbedenkliche Form davon ist und der PDE-Hemmer ein PDE-3-, PDE-4- oder PDE-5-Hemmer ist.

3. Zusammensetzung zur Verwendung nach Anspruch 2, wobei der PDE-Hemmer ein PDE-5-Hemmer ist.

4. Zusammensetzung zur Verwendung nach Anspruch 3, wobei das Tramadol-Material (±)*cis*-Tramadol oder ein pharmazeutisch unbedenkliches Salz davon ist und der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

5. Zusammensetzung zur Verwendung nach Anspruch 4, wobei das Tramadol-Material (±)*cis*-Tramadolhydrochlorid ist und der PDE-5-Hemmer Sildenafilcitrat, Vardenafilhydrochlorid, Tadalafil oder eine Kombination von zwei oder mehr der vorstehenden PDE-5-Hemmer ist.

6. Zusammensetzung zur Verwendung nach Anspruch 5, wobei der PDE-5-Hemmer Sildenafilcitrat ist.

7. Verwendung einer Zusammensetzung, die einen pharmazeutisch unbedenklichen Trägerstoff und Wirkstoffe umfasst, wobei die Wirkstoffe aus einem Tramadol-Material und einem Phosphodiesterase-Hemmer (PDE-Hemmer) bestehen, bei der Herstellung eines Arzneimittels zur Behandlung von komorbidem vorzeitigem Samenerguss und erektiler Dysfunktion bei einem Mann, das eine wirksame Zeit vor sexueller Aktivität zu verabreichen ist.

8. Verwendung nach Anspruch 7, wobei das Tramadol-Material Tramadol oder eine pharmazeutisch unbedenkliche Form davon ist und der PDE-Hemmer ein PDE-3-, PDE-4- oder PDE-5-Hemmer ist.

9. Verwendung nach Anspruch 8, wobei der PDE-Hemmer ein PDE-5-Hemmer ist.

10. Verwendung nach Anspruch 9, wobei das Tramadol-Material (±)cis-Tramadol oder ein pharmazeutisch unbedenkliches Salz davon ist und der PDE-5-Hemmer Sildenafil oder eine pharmazeutisch unbedenkliche Form davon, Vardenafil oder eine pharmazeutisch unbedenkliche Form davon, Tadalafil oder eine pharmazeutisch unbedenkliche Form davon oder eine Kombination von zwei oder mehr der vorstehenden PDE-5-Hemmer ist.

11. Verwendung nach Anspruch 10, wobei das Tramadol-Material (±)cis-Tramadolhydrochlorid ist und der PDE-5-Hemmer Sildenafilcitrat, Vardenafilhydrochlorid, Tadalafil oder eine Kombination von zwei oder mehr der vorstehenden PDE-5-Hemmer ist.

12. Verwendung nach Anspruch 11, wobei der PDE-5-Hemmer Sildenafilcitrat ist.

## Revendications

1. Composition pharmaceutique destinée à être utilisée dans le traitement de l'éjaculation précoce et d'un dysfonctionnement érectile comorbides chez un homme devant être administrée un temps efficace avant l'activité sexuelle, la composition comprenant un véhicule pharmaceutiquement acceptable et des principes actifs, dans laquelle les principes actifs sont constitués d'une substance à base de tramadol et d'un inhibiteur de phosphodiestérase (PDE).

2. Composition destinée à être utilisée selon la revendication 1, dans laquelle la substance à base de tramadol est le tramadol ou une forme pharmaceutiquement acceptable de celui-ci et l'inhibiteur de PDE est un inhibiteur de la PDE3, de la PDE4 ou de la PDE5.

3. Composition destinée à être utilisée selon la revendication 2, dans laquelle l'inhibiteur de PDE est un inhibiteur de la PDE5.

4. Composition destinée à être utilisée selon la revendication 3, dans laquelle la substance à base de tramadol est le (±)-cis-tramadol ou un sel pharmaceutiquement acceptable de celui-ci et l'inhibiteur de la PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci ou une combinaison de deux ou plusieurs des inhibiteurs de la PDE5 ci-dessus.

5. Composition destinée à être utilisée selon la revendication 4, dans laquelle la substance à base de tramadol est le chlorhydrate de (±)-cis-tramadol et l'inhibiteur de la PDE5 est le citrate de sildénafil, le chlorhydrate de vardénafil, le tadalafil ou une combinaison de deux ou plusieurs des inhibiteurs de la PDE5 ci-dessus.

6. Composition destinée à être utilisée selon la revendication 5, dans laquelle l'inhibiteur de la PDE5 est le citrate de sildénafil.

7. Utilisation d'une composition comprenant un véhicule pharmaceutiquement acceptable et des principes actifs, dans laquelle les principes actifs sont constitués d'une substance à base de tramadol et d'un inhibiteur de phosphodiestérase (PDE), dans la préparation d'un médicament pour le traitement de l'éjaculation précoce et d'un dysfonctionnement érectile comorbides chez un homme devant être administré un temps efficace avant l'activité sexuelle.

8. Utilisation selon la revendication 7, dans laquelle la substance à base de tramadol est le tramadol ou une forme pharmaceutiquement acceptable de celui-ci et l'inhibiteur de PDE est un inhibiteur de la PDE3, de la PDE4 ou de la PDE5.

9. Utilisation selon la revendication 8, dans laquelle l'inhibiteur de PDE est un inhibiteur de la PDE5.

10. Utilisation selon la revendication 9, dans laquelle la substance à base de tramadol est le (±)-cis-tramadol ou un sel pharmaceutiquement acceptable de celui-ci et l'inhibiteur de la PDE5 est le sildénafil ou une forme pharmaceutiquement acceptable de celui-ci, le vardénafil ou une forme pharmaceutiquement acceptable de celui-ci, le tadalafil ou une forme pharmaceutiquement acceptable de celui-ci ou une combinaison de deux ou plusieurs des inhibiteurs de la PDE5 ci-dessus.

11. Utilisation selon la revendication 10, dans laquelle la substance à base de tramadol est le chlorhydrate de (±)-cis-tramadol et l'inhibiteur de la PDE5 est le citrate de sildénafil, le chlorhydrate de vardénafil, le tadalafil ou une combinaison de deux ou plusieurs des inhibiteurs de la PDE5 ci-dessus.

12. Utilisation selon la revendication 11, dans laquelle l'inhibiteur de la PDE5 est le citrate de sildénafil.
